# FASCICULE DE BREVET EUROPEEN

(11) **EP 3 347 060 B1**
(45) Date de publication et mention de la délivrance du brevet: **29.06.2022**
(21) Numéro de dépôt: 16778430.5
(22) Date de dépôt: 09.09.2016
(51) Int. Cl.: A61L 2/26, A61C 19/00, A61B 50/20, A61B 50/22

(54) **SYSTEME DE SUPPORT POUR INSTRUMENTS MEDICAUX A STERILISER OU A NETTOYER**
SYSTEM ZUM TRAGEN MEDIZINISCHER, ZU STERILISIERENDER ODER REINIGENDER INSTRUMENTE
SYSTEM FOR SUPPORTING MEDICAL INSTRUMENTS THAT ARE TO BE STERILIZED OR CLEANED

(30) Priorité: 10.09.2015 FR 1558441
(43) Date de publication de la demande: 18.07.2018
(73) Titulaire: Applications Industrielles des Plastiques AIP, 69680 Chassieu (FR)
(72) Inventeur: BEISSMANN, Ludovic, 01300 Cuzieu (FR); COLLET-BEILLON, Brice, 69230 Saint Genis Laval (FR)
(74) Mandataire: Cabinet Beau de Loménie
(86) Numéro de dépôt international: PCT/FR2016/052256
(87) Numéro de publication internationale: WO 2017/042502

(56) Documents cités:
- WO-A1-2006/071180
- WO-A1-2012/084199
- WO-A1-2013/025983
- WO-A1-2014/188348
- US-A1- 2013 046 289

## Description

La présente invention concerne le domaine technique du nettoyage et/ou de la stérilisation d'instruments médicaux comportant, en particulier, un élément de fixation tel qu'une quille, une queue ou une tige apte à pouvoir être maintenue à travers un trou lors de l'opération de nettoyage ou de stérilisation.

L'objet de l'invention vise plus précisément les équipements de conditionnement pour la stérilisation ou le nettoyage d'instruments médicaux comme par exemple les implants en particuliers dentaires ou orthopédiques, et les instruments chirurgicaux.

Dans le domaine d'application préféré de l'invention, il apparait le besoin de pouvoir nettoyer et/ou stériliser de tels instruments médicaux. A cet effet, les instruments médicaux sont maintenus classiquement à l'aide d'un système de support permettant la manutention et le positionnement des instruments médicaux à l'intérieur des machines à stériliser et/ou à nettoyer.

Dans l'état de la technique, il est connu différents systèmes de support tels que des plateaux, des conteneurs ou des boîtes, réalisés en matière plastique ou métallique. Un système de support comporte généralement une plaque présentant une face interne et une face externe, et dans laquelle est aménagée, classiquement, une série de trous adaptés chacun pour recevoir un élément de fixation d'un instrument médical introduit à partir de la face externe de la plaque. Chaque instrument médical est ainsi maintenu en position lors des diverses opérations de manutention ayant pour objectif leur nettoyage ou stérilisation mais également pendant l'opération proprement dite de nettoyage ou de stérilisation. Un tel système de support doit garantir un maintien des instruments médicaux lors de ce processus de nettoyage ou de stérilisation tout en limitant les surfaces de contact avec l'instrument afin de garantir un bon nettoyage ou stérilisation.

Il est connu dans l'état de la technique, de nombreuses solutions pour maintenir les instruments médicaux lors de ce processus de nettoyage et garantir un bon nettoyage ou stérilisation. Une solution consiste à équiper chaque trou de la plaque support, d'un œillet réalisé en un matériau souple ou flexible, pour retenir par friction, l'instrument médical. Dans le cas où les œillets restent montés de façon permanente à la plaque support, il apparaît une difficulté à obtenir un nettoyage ou une stérilisation efficace du système de support, en particulier au niveau de la jonction entre l'œillet et la plaque support.

Afin de garantir un bon nettoyage ou une stérilisation efficace, les documents WO 2013/040363 et WO 2013/040380 proposent de munir chaque œillet, d'un système de liaison démontable avec la plaque. D'une manière générale, chaque œillet comporte un fût tubulaire pourvu à une extrémité, d'un rebord prenant appui sur la face externe de la plaque et à son autre extrémité, d'un bourrelet venant se bloquer sur la face interne de la plaque. En pratique, les opérations de montage et démontage conduisent à une dégradation de l'œillet susceptible de créer des zones difficiles à nettoyer ou à stériliser.

Les documents WO2006/071180, US2013/0046289 et WO2013/025983 décrivent des systèmes de support pour instruments médicaux à oeillets, présentant les mêmes caractéristiques et les mêmes inconvénients de nettoyage et de stérilisation que les dispositifs décrits ci-devant.

La demande de brevet WO 2012/084 199 décrit une cassette pour maintenir des instruments médicaux comportant une plaque pourvue de trous et une structure de maintien élastique montée sur la plaque par une liaison par encliquetage. Cette structure de maintien élastique est montée pour être située à distance de la surface supérieure de la plaque et de manière que le logement de maintien d'un instrument se trouve formé en partie haute, par le trou de la plaque et en partie basse, par un alésage aménagé dans la structure de maintien élastique. Un inconvénient de ce système concerne la difficulté à obtenir un nettoyage ou une stérilisation efficace en particulier au niveau de l'interface entre la plaque et la structure de maintien élastique.

Il est également connu notamment par le document WO 2014/089420, de réaliser le système de support par une feuille en matière souple interposée entre une plaque et une contre plaque assemblées ensemble. La plaque et la contre plaque ainsi que la feuille en matière souple sont pourvues de trous aménagés en coïncidence pour permettre le maintien des instruments médicaux. Cette solution présente un coût non négligeable en raison de l'utilisation d'une feuille en matière souple.

La demande de brevet WO2014/188348 décrit un dispositif de support d'instruments médicaux comportant un support formé par une couche de support dans laquelle est aménagée une série de trous traversants s'étendant de la surface avant à la surface arrière. Chaque trous est pourvu d'une couche de recouvrement positionnée uniquement au niveau de chaque trou de la couche de support et destiné à assurer le maintien d'un élément de fixation d'un instrument médical. La couche de support et la couche de recouvrement sont liées entre elles pour former un corps unique.

La présente invention vise à remédier aux inconvénients de l'état de la technique en proposant un nouveau système de support pour instruments médicaux à stériliser et/ou à nettoyer, conçu pour améliorer le nettoyage et/ou la stérilisation des instruments médicaux tout en étant de conception simple en présentant un coût réduit.

Pour atteindre un tel objectif, le système de support d'instruments médicaux à stériliser et/ou à nettoyer comporte une plaque présentant une face interne et une face externe et dans laquelle est aménagé au moins un trou traversant de montage pour une structure souple de maintien pour l'élément de fixation d'un instrument médical, cette structure souple comportant un système de liaison démontable avec la plaque, chaque trou de montage comportant un système de liaison démontable complémentaire du système de liaison de la structure souple, ce système de liaison démontable complémentaire étant aménagé exclusivement à partir de la paroi intérieure du trou de montage délimitée entre la face interne et la face externe de la plaque. Selon l'invention, la structure souple comporte une surface supérieure affleurant avec la face externe de la plaque et une surface inférieure affleurant avec la face interne de la plaque.

De plus, le système selon l'invention peut comporter en outre en combinaison au moins l'une et/ou l'autre des caractéristiques additionnelles suivantes :
- le système de liaison démontable complémentaire aménagé exclusivement à partir de la paroi intérieure du trou de montage comporte au moins une surface d'arrêt de la structure souple de maintien, selon au moins un sens de montage de ladite structure souple de maintien, à partir de la face externe de la plaque,
- le système de liaison démontable complémentaire aménagé exclusivement à partir de la paroi intérieure du trou de montage comporte des surfaces d'arrêt de la structure souple de maintien, selon les deux sens de montage de ladite structure souple de maintien, à partir des deux faces de la plaque,
- le système de liaison démontable complémentaire du système de liaison démontable de la structure souple est du type à emboîtement, vissage ou quart de tour,
- la structure souple comporte au moins un pion monté dans un trou de la plaque et présentant une surface latérale externe pourvue du système de liaison démontable, ce pion débouchant latéralement en partie dans un passage de réception et de maintien par coincement, d'un élément de fixation d'un instrument médical, ce passage de réception et de maintien étant aménagé dans la plaque,
- le pion comporte en tant que système de liaison démontable, une partie terminale de section réduite par rapport à la section du pion, cette partie terminale coopérant avec un épaulement annulaire aménagé dans la paroi intérieure du trou de la plaque,
- la structure souple comporte un œillet présentant dans sa partie centrale, un passage de réception et de maintien par coincement, d'un élément de fixation d'un instrument médical, cet œillet présentant une surface latérale externe pourvue du système de liaison démontable,
- l'œillet comporte, sur sa surface latérale externe en tant que système de liaison démontable, une nervure annulaire coopérant avec un logement de forme complémentaire aménagé dans la paroi intérieure du trou de montage,
- l'œillet comporte, sur sa surface latérale externe en tant que système de liaison démontable, une portion tronconique s'étendant à partir de la surface supérieure jusqu'à une paroi radiale destinée à coopérer avec une surface d'arrêt du système de liaison démontable complémentaire,
- l'œillet comporte, sur sa surface latérale externe en tant que système de liaison démontable, un filet de pas de vis coopérant avec un taraudage du trou traversant,
- l'œillet comporte, sur sa surface latérale externe en tant que système de liaison démontable, au moins un ergot destiné à être engagé à travers une lumière communiquant avec une cavité de blocage destinée à recevoir l'ergot après rotation de l'œillet.

Un autre objet de l'invention est de proposer un équipement de conditionnement pour la stérilisation et/ou le nettoyage d'instruments médicaux à éléments de fixation comprenant au moins un dispositif de support conforme à l'invention.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue de ¾ de dessus en perspective montrant divers exemples de réalisation du système de support conforme à l'invention, maintenant en position des instruments médicaux.
La **Figure 2** est une vue de ¾ de dessous en perspective du système de support illustré à la **Fig. 1****.**
La **Figure 3** est une vue du système de support analogue à la **Fig. 1** mais illustré sans instruments médicaux.
La **Figure 4** est une vue en perspective analogue à la **Fig. 3** montrant les structures de maintien des instruments médicaux avant leur montage.
La **Figure 5** est une vue en coupe élévation du système de support prises selon les lignes V-V de la **Fig. 3****.**
La **Figure 6** est une vue en perspective en partie arrachée, montrant des détails caractéristiques du système de support conforme à l'invention.
La **Figure 7** est une vue en coupe élévation montrant une variante de réalisation de la structure de maintien des instruments médicaux.

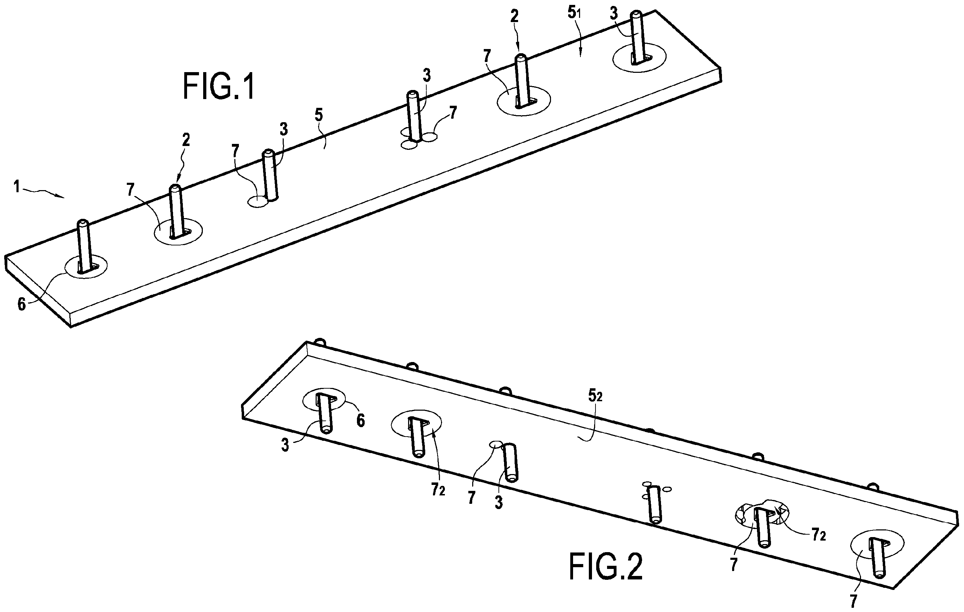

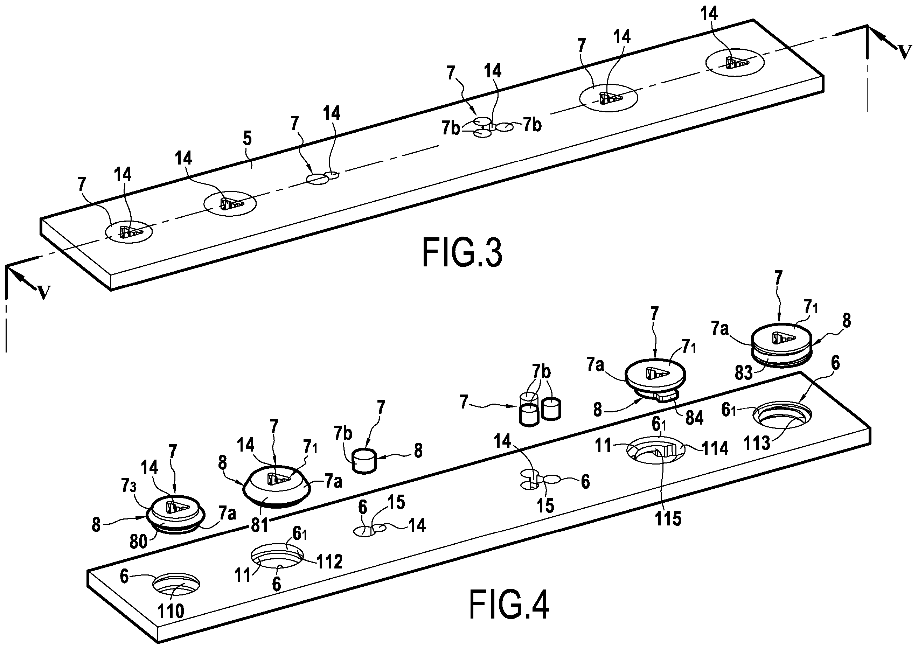

Tel que cela ressort plus précisément des **Fig. 1** à **7****,** l'objet de l'invention concerne un dispositif de support **1** pour des instruments médicaux **2** à nettoyer et/ou à stériliser de tout type connu en soi mais non représentés de manière complète. D'une manière générale, chaque instrument médical **2** comporte un élément de fixation **3** tel qu'une quille, une queue ou une tige apte à pouvoir être maintenue à travers un trou lors de l'opération de nettoyage ou de stérilisation. Les instruments médicaux **2** sont par exemple des implants en particuliers dentaires ou orthopédiques ou des instruments chirurgicaux. Il est à noter que l'instrument médical **2** a été représenté sur les dessins essentiellement par un élément de fixation **3** uniquement à titre d'illustration non limitative.

Le dispositif de support **1** selon l'invention comporte une plaque **5** présentant au moins un, et dans l'exemple illustré, plusieurs trous **6** aménagés chacun pour assurer le montage d'une structure souple ou flexible **7** de maintien pour l'élément de fixation **3** d'un instrument médical. La plaque **5** présente une première face dite externe ou supérieure **5₁** et une deuxième face dite interne ou inférieure **5₂**, prises en considération du sens de positionnement de l'instrument médical **3** par rapport à la plaque. Ainsi, il est à considérer que l'élément de fixation **3** d'un instrument médical est maintenu par une structure souple **7** en étant introduit (mais aussi retiré) à partir de la face supérieure **5₁** de la plaque.

Chaque trou **6** est traversant c'est-à-dire qu'il débouche sur la face supérieure **5₁** et la face inférieure **5₂**, en présentant une paroi intérieure **6₁** délimitée entre la face supérieure **5₁** et la face inférieure **5₂**. Chaque trou **6** présente un axe longitudinal de symétrie **X** sensiblement perpendiculaire à la face supérieure **5₁** et la face inférieure **5₂**.

La plaque **5** est réalisée en tout matériau approprié apte à supporter une opération de nettoyage et/ou de stérilisation. Par exemple, la plaque **5** est réalisée en matière plastique ou en matériau métallique. Dans l'exemple illustré, la plaque **5** présente des trous **6** aménagés selon une seule rangée mais il est clair que d'une manière générale, la plaque présente une série de trous pour la réception et le support de plusieurs instruments médicaux, répartis de toute manière appropriée selon la surface de la plaque. Le dispositif de support **1** se présente donc sous la forme d'une plaque avec une forme et des dimensions adaptées aux applications visées. Il est à noter que la plaque **5** peut être aménagée pour recevoir des systèmes de support différents de l'objet de l'invention pour le maintien d'instruments médicaux complémentaires de ceux maintenus par la structure souple **7.** D'une manière générale, le dispositif de support **1** fait partie d'un équipement de conditionnement pour la stérilisation et le nettoyage d'instruments médicaux se présentant sous la forme d'une plaque ou de plateaux, intégrés ou non à l'intérieur de boîtes ou de kits de nettoyage ou de stérilisation.

La structure souple **7** est réalisée en un matériau souple ou flexible c'est-à-dire présentant une capacité à se déformer élastiquement, de manière réversible. Typiquement, la structure souple **7** est réalisée en tous matériaux appropriés adaptés pour supporter une opération de nettoyage et/ou de stérilisation.

Cette structure souple **7** comporte un système **8** de liaison démontable avec la plaque **5.** En d'autres termes, ce système **8** assure une liaison ou un assemblage démontable entre la structure souple **7** et la plaque **5.** Comme cela sera décrit plus en détail dans la suite de la description, la structure souple **7** qui présente un caractère amovible, peut ainsi être fixée de manière temporaire à la plaque **5** et être démontée à volonté sans entrainer une détérioration de cette structure souple ni de la plaque. Typiquement, la structure souple **7** est montée sur la plaque **5** selon au moins un sens de montage à partir d'une face, de préférence, la face supérieure **5₁** de la plaque. Bien entendu, la structure souple **7** peut être montée sur la plaque **5** selon deux sens de montage à savoir indifféremment à partir de la face supérieure **5₁** ou de la face inférieure **5₂**.

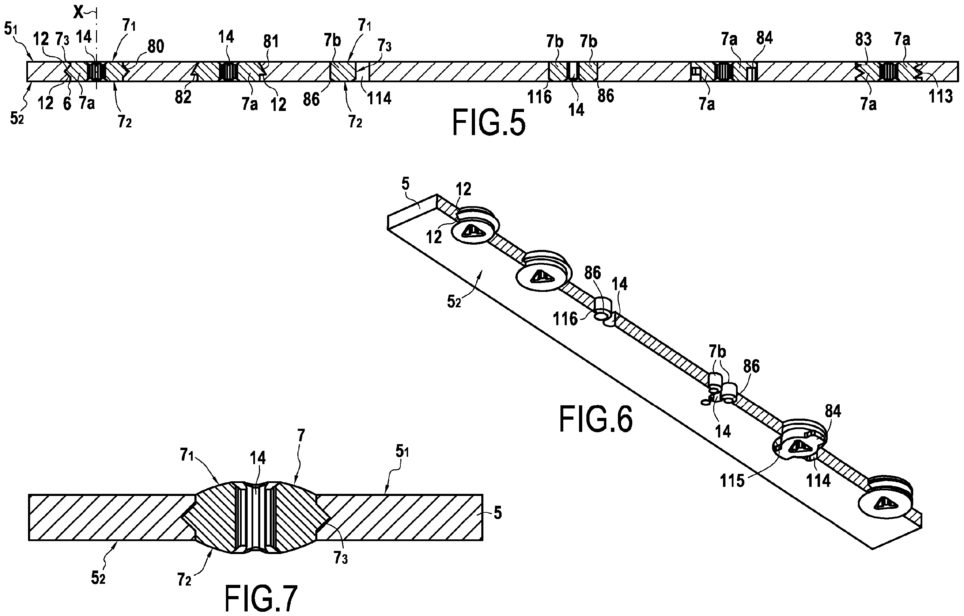

La structure souple **7** comporte entre une surface supérieure **7₁** et une surface inférieure **7₂**, une surface latérale externe **7₃**. Selon une caractéristique de l'invention, en position montée de la structure souple **7,** la surface supérieure **7₁** est affleurant avec la face supérieure **5₁** de la plaque **5** et la surface inférieure **7₂** est affleurant avec la face inférieure **5₂** de la plaque 5. Ainsi, tel que cela ressort clairement des **Fig. 1** à **6****,** la surface supérieure **7₁** et la surface inférieure **7₂** s'étendent dans des plans confondus avec les plans contenant respectivement la face supérieure **5₁** et la face inférieure **5₂**. Toutefois, tel que cela ressort de l'exemple illustré à la **Fig. 7****,** la surface supérieure **7₁** et la surface inférieure **7₂** de la structure souple **7** peuvent présenter une forme non plane par exemple bombée tout en restant affleurant ou au même niveau avec les faces externe et interne de la plaque **5.** En effet, chaque bord périphérique délimité par la jonction entre la surface latérale externe **7₃** et une surface inférieure **7₂** ou supérieure **7₁** est situé au niveau d'une face de la plaque. Il n'apparaît donc pas d'épaulement au niveau de la jonction entre la structure souple **7** et la plaque **5.** En d'autres termes, la hauteur de la surface latérale externe **7₃** de la structure souple possède une hauteur identique à l'épaisseur de la plaque **5** prise au niveau d'un trou **6.**

Chaque trou de montage **6** comporte un système de liaison démontable **11** qui est complémentaire du système de liaison démontable **8** de la structure souple **7,** ce système de liaison démontable complémentaire **11** étant aménagé exclusivement à partir de la paroi intérieure **6₁** du trou de montage **6.** En d'autres termes, ce système de liaison démontable complémentaire **11** prend place exclusivement sur la paroi intérieure **6₁** du trou, en n'étant pas réalisé sur la face supérieure **5₁** et ni sur la face inférieure **5₂** de la plaque **5.** Il doit être compris qu'une structure souple de maintien **7** et la paroi intérieure **6₁** d'un trou de montage sont pourvues des systèmes de liaison démontables **8, 11** dont l'un est complémentaire de l'autre. Ces systèmes de liaison démontable **8**, **11** sont complémentaires entre eux dans le sens où ils coopèrent entre eux avec des formes géométriques présentant une congruence ou une complémentarité assurant un assemblage amovible entre la structure souple de maintien **7** et la plaque **5.** Réciproquement, le système de liaison démontable **8** de la structure souple **7** est aménagé exclusivement à partir de la surface latérale externe **7₃** de cette structure souple.

Le système de liaison démontable complémentaire **11** comporte au moins une surface d'arrêt **12** de la structure souple de maintien **7,** selon au moins un sens de montage de ladite structure souple de maintien. Cette surface d'arrêt **12** qui est réalisée à partir de la paroi intérieure **6₁** du trou, limite ainsi l'introduction de la structure souple de maintien **7** à l'intérieur de la paroi intérieure **6₁** du trou de montage, à partir d'une face et de préférence, de la face supérieure **5₁** de la plaque.

De préférence, le système de liaison démontable complémentaire **11** comporte deux surfaces d'arrêt **12** pour la structure souple de maintien **7,** selon les deux sens de montage de ladite structure souple de maintien sur la plaque c'est-à-dire à partir de la face externe de la plaque et de la face interne de la plaque.

Chaque surface d'arrêt **12** constitue ainsi une surface de blocage pour la structure souple de maintien **7,** selon l'un ou les deux sens de la direction perpendiculaire aux faces de la plaque c'est-à-dire parallèle à l'axe longitudinal de symétrie **X** du trou **6.** Ainsi, comme cela sera expliqué dans la suite de la description, lors du montage de la structure souple de maintien **7** à l'intérieur du trou **6,** la structure souple de maintien **7** vient en butée sur cette surface d'arrêt **12** lors de son déplacement parallèlement à l'axe longitudinal de symétrie **X** du trou **6.** Ainsi, cette surface d'arrêt **12** s'étend dans un plan sécant à l'axe longitudinal de symétrie **X** du trou **6.**

La suite de la description décrit deux modes de réalisation préférentiels, de la structure souple de maintien **7.** Selon un premier mode de réalisation, la structure souple de maintien **7** se présente sous la forme d'un œillet c'est-à-dire d'un corps tubulaire **7ₐ** pourvu dans sa partie centrale, d'un passage **14** de réception et de maintien par coincement, d'un élément de fixation **3** d'un instrument médical. Chaque passage **14** d'un œillet **7ₐ** se présente sous la forme d'un trou débouchant sur les surfaces supérieure **7₁** et inférieure **7₂** et présentant une configuration adaptée pour maintenir, un élément de fixation **3,** par coincement en raison du caractère souple ou déformable de l'œillet **7ₐ**. Ce passage **14** de l'œillet **7ₐ** est par exemple lisse ou pourvu de nervures ou de pattes élastiques.

Cet œillet **7ₐ** présente une surface latérale externe **7₃** qui est aménagée pour présenter le système de liaison démontable **8.** Tel que cela ressort des **Figures,** les systèmes de liaison démontable **8, 11** entre la structure souple de maintien **7** et la plaque **5** peuvent relever de différents modes d'assemblage ou de liaison, du type à emboîtement, vissage ou quart de tour dans les exemples illustrés sur les dessins.

Selon une variante de réalisation, l'œillet **7ₐ** comporte, sur sa surface latérale externe **7₃** en tant que système de liaison démontable **8,** une nervure annulaire **80** coopérant avec un logement **110** de forme complémentaire aménagé dans la paroi intérieure **6₁** du trou de montage **6.** Par exemple, l'œillet **7ₐ** comporte une nervure annulaire **80** de section droite triangulaire s'étendant en saillie à partir de la surface latérale externe **7₃** de l'œillet et engagée à l'intérieur d'un logement ou d'une rainure annulaire **110** de section droite triangulaire. Selon cet exemple, cette rainure annulaire **110** du système de liaison démontable complémentaire **11** délimite deux surfaces d'arrêt **12** pour la structure souple de maintien **7.** Ainsi, le montage de l'œillet **7ₐ** sur la plaque **5** est réalisé par emboîtement. Bien entendu, la nervure est aménagée dans la paroi intérieure **6₁** du trou de montage **6.**

Selon une autre variante de réalisation, l'œillet **7ₐ** comporte, sur sa surface latérale externe **7₃** en tant que système de liaison démontable **8,** une portion tronconique **81** s'étendant à partir de la surface supérieure **7₁** jusqu'à une paroi radiale **82** destinée à coopérer avec une surface d'arrêt **12** du système de liaison démontable complémentaire **11,** aménagée dans la paroi intérieure **6₁** du trou **6.** Cette portion tronconique **81** coopère avec un logement tronconique **112** de forme complémentaire à la portion tronconique **81,** et aménagé dans la paroi intérieure **6₁** du trou de montage **6.** Ainsi, le montage de l'œillet **7ₐ** sur la plaque **5** est réalisé par emboîtement.

Selon une autre variante de réalisation, l'œillet **7ₐ** comporte, sur sa surface latérale externe **7₃** en tant que système de liaison démontable **8,** un filet de pas de vis **83** coopérant avec un taraudage **113** du trou traversant **6.** Ainsi, le montage de l'œillet **7ₐ** sur la plaque **5** est réalisé par vissage. Selon cette variante, la structure souple **7** est en butée sur deux surfaces d'arrêt **12** formées par les filets du taraudage **113.**

Selon une autre variante de réalisation, l'œillet **7ₐ** comporte, sur sa surface latérale externe **7₃** en tant que système de liaison démontable **8,** au moins un et dans l'exemple illustré, deux ergots **84** destinés à être engagés chacun à travers une lumière **114** débouchant au moins sur la face supérieure **5₁** de la plaque. Chaque lumière **114** est aménagée dans la paroi intérieure **61** du trou de montage **6.** Chaque lumière **114** communique avec une cavité de blocage **115** destinée à recevoir un ergot **84** après rotation de l'œillet. Chaque cavité de blocage **115** est aménagée dans la paroi intérieure **6₁** du trou de montage en présentant une section décroissante pour bloquer l'ergot en fin de rotation de l'œillet. Ainsi, le montage de l'œillet **7ₐ** sur la plaque 5 est réalisé par un assemblage quart de tour.

Selon le premier mode de réalisation décrit ci-dessus, la structure souple de maintien **7** se présente sous la forme d'un oeillet **7ₐ** assurant par lui-même, le maintien de l'élément de fixation **3** d'un instrument médical. Selon un deuxième mode de réalisation, la structure souple **7** assure de manière indirecte, le maintien de l'élément de fixation **3** d'un instrument médical.

Selon ce deuxième mode de réalisation, la structure souple **7** comporte au moins un pion **7_{b}** monté dans un trou **6** de la plaque **5** et présentant une surface latérale externe **7₃** pourvue du système de liaison démontable **8.** Ce pion **7_{b}** débouche latéralement en partie dans un passage **14** de réception et de maintien par coincement, d'un élément de fixation **3** d'un instrument médical. Ce passage de réception et de maintien **14** tel qu'un trou est aménagé dans la plaque **5** en la traversant de part en part, en étant sécant avec un trou **6** pour communiquer ensemble selon un passage d'intercommunication **15.** Il doit être compris qu'une partie de la surface latérale externe **7₃** du pion **7_{b}** débouche dans le trou **14,** par le passage d'intercommunication **15** pour venir en appui ou en contact avec un élément de fixation **3,** et exercer un effort pour maintenir ce dernier en place à l'intérieur du trou **14.**

Selon un exemple de réalisation, la structure souple **7** comporte un pion **7_{b}** monté par emboîtement dans un trou **6** de la plaque **5** tandis que selon un autre exemple de réalisation, la structure souple **7** comporte trois pions **7_{b}** montés chacun par emboîtement dans un trou **6** de la plaque **5** et disposés à 120° les uns des autres autour du passage **14,** en débouchant chacun latéralement dans ce passage central **14.** Chaque pion **7_{b}** comporte en tant que système de liaison démontable **8,** un corps de section ajustée avec le trou **6** pour assurer un assemblage par emboîtement, ce corps comportant une partie terminale **86** de section réduite par rapport à la section du corps du pion. Cette partie terminale **86** coopère avec un épaulement annulaire **116** aménagé dans la paroi intérieure du trou **6** de la plaque **5,** à partir de la face inférieure **5₂** de la plaque **5** et forme une surface d'arrêt **12**.

Le dispositif de support selon l'invention présente l'avantage de ne pas présenter de zones difficiles à nettoyer ou à stériliser dans la mesure où la structure souple **7** est montée de niveau ou en affleurement avec au moins les bords des trous **6** de la plaque. De plus, la structure souple **7** de maintien des instruments médicaux est démontable, ce qui permet de pouvoir changer ces structures souples de maintien tout en autorisant un nettoyage ou stérilisation efficace du dispositif de support selon l'invention.

L'invention n'est pas limitée aux exemples décrits et représentés car diverses modifications peuvent y être apportées sans sortir de son cadre.

## Revendications

1. - Dispositif de support d'instruments médicaux **(2)** à stériliser et/ou à nettoyer comportant chacun un élément de fixation **(3),** le dispositif de support **(1)** comportant une plaque **(5)** présentant une face inférieure (**5₂**) et une face supérieure (**5₁**) et dans laquelle est aménagé au moins un trou traversant **(6)** de montage pour une structure souple **(7)** de maintien pour l'élément de fixation **(3)** d'un instrument médical, cette structure souple **(7)** comportant un système **(8)** de liaison démontable avec la plaque **(5),** chaque trou de montage **(6)** comportant un système **(11)** de liaison démontable complémentaire du système de liaison **(8)** de la structure souple, ce système de liaison démontable complémentaire **(11)** étant aménagé exclusivement à partir de la paroi intérieure (**6₁**) du trou de montage délimitée entre la face inférieure (**5₂**) et la face supérieure (**5₁**) de la plaque, **caractérisé en ce que** la structure souple **(7)** comporte une surface supérieure (**7₁**) affleurant avec la face supérieure (**5₁**) de la plaque **(5)** et **en ce que** la structure souple **(7)** comporte une surface inférieure (**7₂**) affleurant avec la face inférieure (**5₂**) de la plaque **(5).**

2. - Dispositif de support selon la revendication 1, **caractérisé en ce que** le système de liaison démontable complémentaire **(11)** aménagé exclusivement à partir de la paroi intérieure du trou de montage **(6)** comporte au moins une surface d'arrêt **(12)** de la structure souple de maintien **(7),** selon au moins un sens de montage de ladite structure souple de maintien, à partir de la face supérieure (**5₁**) de la plaque.

3. - Dispositif de support selon la revendication 2, **caractérisé en ce que** le système de liaison démontable complémentaire **(11)** aménagé exclusivement à partir de la paroi intérieure du trou de montage **(6)** comporte des surfaces d'arrêt **(12)** de la structure souple de maintien **(7),** selon les deux sens de montage de ladite structure souple de maintien, à partir des deux faces de la plaque.

4. - Dispositif de support selon l'une des revendications 1 à 3, **caractérisé en ce que** le système de liaison démontable complémentaire (**11**) du système de liaison démontable **(8)** de la structure souple **(7)** est du type à emboîtement.

5. - Dispositif de support selon l'une des revendications 1 à 3, **caractérisé en ce que** le système de liaison démontable complémentaire **(11)** du système de liaison démontable **(8)** de la structure souple **(7)** est du type à vissage.

6. - Dispositif de support selon l'une des revendications 1 à 3, **caractérisé en ce que** le système de liaison démontable complémentaire **(11)** du système de liaison démontable **(8)** de la structure souple **(7)** est du type à quart de tour.

7. - Dispositif de support selon l'une des revendications 1 à 6, **caractérisé en ce que** la structure souple **(7)** comporte au moins un pion **(7b)** monté dans un trou **(6)** de la plaque **(5)** et présentant une surface latérale externe pourvue du système de liaison démontable **(8),** ce pion **(7b)** débouchant latéralement en partie dans un passage **(14)** de réception et de maintien par coincement, d'un élément de fixation **(3)** d'un instrument médical, ce passage de réception et de maintien **(14)** étant aménagé dans la plaque **(5).**

8. - Dispositif de support selon la revendication 7, **caractérisé en ce que** le pion (**7b**) comporte en tant que système de liaison démontable **(8),** une partie terminale **(86)** de section réduite par rapport à la section du pion, cette partie terminale **(86)** coopérant avec un épaulement annulaire **(116)** aménagé dans la paroi intérieure du trou **(6)** de la plaque **(5).**

9. - Dispositif de support selon l'une des revendications 1 à 6, **caractérisé en ce que** la structure souple **(7)** comporte un oeillet **(7a)** présentant dans sa partie centrale, un passage **(14)** de réception et de maintien par coincement, d'un élément de fixation **(3)** d'un instrument médical, cet œillet **(7a)** présentant une surface latérale externe (**7₃**) pourvue du système de liaison démontable.

10. - Dispositif de support selon la revendication 9, **caractérisé en ce que** l'œillet **(7a)** comporte, sur sa surface latérale externe (**7₃**) en tant que système de liaison démontable **(8),** une nervure annulaire **(80)** coopérant avec un logement **(110)** de forme complémentaire aménagé dans la paroi intérieure (**6₁**) du trou de montage.

11. - Dispositif de support selon la revendication 9, **caractérisé en ce que** l'œillet **(7a)** comporte, sur sa surface latérale externe (**7₃**) en tant que système de liaison démontable (**8**), une portion tronconique(**81**) s'étendant à partir de la surface supérieure **(82)** jusqu'à une paroi radiale destinée à coopérer avec une surface d'arrêt **(12)** du système de liaison démontable complémentaire **(11).**

12. - Dispositif de support selon la revendication 9, **caractérisé en ce que** l'œillet **(7a)** comporte, sur sa surface latérale externe (**7₃**) en tant que système de liaison démontable **(8),** un filet de pas de vis **(83)** coopérant avec un taraudage **(113)** du trou traversant **(6).**

13. - Dispositif de support selon la revendication 9, **caractérisé en ce que** l'œillet **(7a)** comporte, sur sa surface latérale externe (**7₃**) en tant que système de liaison démontable **(8),** au moins un ergot **(84)** destiné à être engagé à travers une lumière **(114)** communiquant avec une cavité de blocage **(115)** destinée à recevoir l'ergot après rotation de l'œillet.

14. - Equipement de conditionnement pour la stérilisation et/ou le nettoyage d'instruments médicaux **(2)** à éléments de fixation **(3), caractérisé en ce qu'**il comporte au moins un dispositif de support (**1**) conforme à l'une des revendications 1 à 13.

## Patentansprüche

1. Vorrichtung zum Halten von zu sterilisierenden und/oder zu reinigenden medizinischen Instrumenten (2), die jeweils ein Befestigungselement (3) umfassen, wobei die Haltevorrichtung (1) eine Platte (5) umfasst, welche eine Unterseite (5₂) und einer Oberseite (5₁) aufweist und in der wenigstens ein Durchgangsloch (6) zum Montieren einer flexiblen Struktur (7) zum Halten des Befestigungselements (3) eines medizinischen Instruments angeordnet ist, wobei diese flexible Struktur (7) ein System (8) zum lösbaren Verbinden mit der Platte (5) umfasst, wobei jedes Montageloch (6) ein System (11) zum lösbaren Verbinden umfasst, das komplementär zu dem Verbindungssystem (8) der flexiblen Struktur ist, wobei dieses komplementäre System zum lösbaren Verbinden (11) ausschließlich von der Innenwand (6₁) des Montagelochs aus, welche zwischen der Unterseite (5₂) und der Oberseite (5₁) der Platte begrenzt ist, angeordnet ist, **dadurch gekennzeichnet, dass** die flexible Struktur (7) eine obere Fläche (7₁) aufweist, die mit der Oberseite (5₁) Platte (5) bündig abschließt und dass die flexible Struktur (7) eine untere Fläche (7₂) umfasst, die mit der Unterseite (5₂) der Platte (5) bündig abschließt.

2. Haltevorrichtung nach Anspruch 1, **dadurch gekennzeichnet, dass** das komplementäre System zum lösbaren Verbinden (11), das ausschließlich von der Innenwand des Montagelochs (6) aus angeordnet ist, wenigstens eine Fläche zum Arretieren (12) der flexiblen Haltestruktur (7) in wenigstens einer Montagerichtung der flexiblen Haltestruktur, von der Oberseite (5₁) der Platte aus umfasst.

3. Haltevorrichtung nach Anspruch 2, **dadurch gekennzeichnet, dass** das komplementäre System zum lösbaren Verbinden (11), das ausschließlich von der Innenwand des Montagelochs (6) aus angeordnet ist, Flächen zum Arretieren (12) der flexiblen Haltestruktur (7) in beiden Montagerichtungen der flexiblen Haltestruktur, von beiden Seiten der Platte aus umfasst.

4. Haltevorrichtung nach einem der Ansprüche 1 bis 3, **dadurch gekennzeichnet, dass** das System zum lösbaren Verbinden (11), welches zu dem System zum lösbaren Verbinden (8) der flexiblen Struktur (7) komplementär ist, vom Einsteck-Typ ist.

5. Haltevorrichtung nach einem der Ansprüche 1 bis 3, **dadurch gekennzeichnet, dass** das System zum lösbaren Verbinden (11), welches zu dem System zum lösbaren Verbinden (8) der flexiblen Struktur (7) komplementär ist, vom Einschraub-Typ ist.

6. Haltevorrichtung nach einem der Ansprüche 1 bis 3, **dadurch gekennzeichnet, dass** das System zum lösbaren Verbinden (11), welches zu dem System zum lösbaren Verbinden (8) der flexiblen Struktur (7) komplementär ist, vom Vierteldrehungs-Typ ist.

7. Haltevorrichtung nach einem der Ansprüche 1 bis 6, **dadurch gekennzeichnet, dass** die flexible Struktur (7) wenigstens einen Stift (7b) umfasst, der in einem Loch (6) der Platte (5) angebracht ist und eine äußere Mantelfläche aufweist, die mit dem System zum lösbaren Verbinden (8) versehen ist, wobei dieser Stift (7b) seitlich teilweise in einen Durchgang (14) zum klemmenden Aufnehmen und Halten eines Befestigungselements (3) eines medizinischen Instruments mündet, wobei dieser Aufnahme- und Haltedurchgang (14) in der Platte (5) ausgebildet ist.

8. Haltevorrichtung nach Anspruch 7, **dadurch gekennzeichnet, dass** der Stift (7b) als System zum lösbaren Verbinden (8) einen Endteil (86) mit reduziertem Querschnitt in Bezug auf den Querschnitt des Stifts umfasst, wobei dieser Endteil (86) mit einer ringförmigen Schulter (116) zusammenwirkt, die in der Innenwand des Lochs (6) der Platte (5) ausgebildet ist.

9. Haltevorrichtung nach einem der Ansprüche 1 bis 6, **dadurch gekennzeichnet, dass** die flexible Struktur (7) eine Öse (7a) umfasst, die in ihrem mittleren Teil einen Durchgang (14) zum klemmenden Aufnehmen und Halten eines Befestigungselements (3) eines medizinischen Instruments aufweist, wobei diese Öse (7a) eine äußere Mantelfläche (7₃) aufweist, die mit dem System zum lösbaren Verbinden versehen ist.

10. Haltevorrichtung nach Anspruch 9, **dadurch gekennzeichnet, dass** die Öse (7a) an ihrer äußeren Mantelfläche (7₃) als System zum lösbaren Verbinden (8) eine ringförmige Rippe (80) aufweist, die mit einer Aufnahme (110) mit komplementärer Form zusammenwirkt, welche in der Innenwand (6₁) des Montagelochs ausgebildet ist.

11. Haltevorrichtung nach Anspruch 9, **dadurch gekennzeichnet, dass** die Öse (7a) an ihrer äußeren Mantelfläche (7₃) als System zum lösbaren Verbinden (8) einen kegelstumpfförmigen Abschnitt (81) aufweist, der sich von der oberen Fläche (82) bis zu einer radialen Wand erstreckt, welche dazu bestimmt ist, mit einer Fläche zum Arretieren (12) des komplementären Systems zum lösbaren Verbinden (11) zusammenzuwirken.

12. Haltevorrichtung nach Anspruch 9, **dadurch gekennzeichnet, dass** die Öse (7a) an ihrer äußeren Mantelfläche (7₃) als System zum lösbaren Verbinden (8) ein Schraubgewinde (83) aufweist, das mit einem Innengewinde (113) des Durchgangslochs (6) zusammenwirkt.

13. Haltevorrichtung nach Anspruch 9, **dadurch gekennzeichnet, dass** die Öse (7a) an ihrer äußeren Mantelfläche (7₃) als System zum lösbaren Verbinden (8) wenigstens einen Nocken (84) aufweist, der dazu bestimmt ist, durch eine Öffnung (114) gesteckt zu werden, die mit einem Blockierhohlraum (115) in Verbindung steht, welcher dazu bestimmt ist, den Nocken nach Drehen der Öse aufzunehmen.

14. Verpackungseinrichtung für die Sterilisation und/oder die Reinigung von medizinischen Instrumenten (2) mit Befestigungselementen (3), **dadurch gekennzeichnet, dass** sie wenigstens eine Haltevorrichtung (1) nach einem der Ansprüche 1 bis 13 umfasst.

## Claims

1. A support device for medical instruments (2) to be sterilized and/or cleaned, each including a fixing element (3), the support device (1) including a plate (5) having a lower face (5₂) and a upper face (5₁) and in which at least one mounting through hole (6) for a flexible holding structure (7) for the medical instrument fixing element (3) is arranged, this flexible structure (7) including a dismountable connection system (8) with the plate (5), each mounting hole (6) including a dismountable connection system (11) complementary to the connection system (8) of the flexible structure, this complementary dismountable connection system (11) being arranged exclusively from the internal wall (6₁) of the mounting hole delimited between the lower face (5₂) and the upper face (5₁) of the plate, **characterized in that** the flexible structure (7) includes an upper surface (7₁) flush with the upper face (5₁) of the plate (5) and **in that** the flexible structure (7) includes a lower surface (7₂) flush with the lower face (5₂) of the plate (5).

2. The support device according to claim 1, **characterized in that** the complementary dismountable connection system (11) arranged exclusively from the internal wall of the mounting hole (6) includes at least one stop surface (12) of the flexible holding structure (7), according to at least one mounting direction of said flexible holding structure, from the upper face (5₁) of the plate.

3. The support device according to claim 2, **characterized in that** the complementary dismountable connection system (11) arranged exclusively from the internal wall of the mounting hole (6) includes stop surfaces (12) of the flexible holding structure (7), along the two mounting directions of said flexible holding structure, from the two faces of the plate.

4. The support device according to any of claims 1 to 3, **characterized in that** the complementary dismountable connection system (11) of the dismountable connection system (8) of the flexible structure (7) is of the interlocking type.

5. The support device according to any of claims 1 to 3, **characterized in that** the complementary dismountable connection system (11) of the dismountable connection system (8) of the flexible structure (7) is of the screwing type.

6. The support device according to any of claims 1 to 3, **characterized in that** the complementary dismountable connection system (11) of the dismountable connection system (8) of the flexible structure (7) is of the quarter-turn type.

7. The support device according to any of claims 1 to 6, **characterized in that** the flexible structure (7) includes at least one pin (7b) mounted in a hole (6) of the plate (5) and having an outer side surface provided with the dismountable connection system (8), this pin (7b) opening out laterally partly into a passage (14) for receiving and holding by wedging, a medical instrument fixing element (3), this receiving and holding passage (14) being arranged in the plate (5).

8. The support device according to claim 7, **characterized in that** the pin (7b) includes, as a dismountable connection system (8), an end part (86) of reduced section compared to the section of the pin, this end part (86) cooperating with an annular shoulder (116) arranged in the internal wall of the hole (6) of the plate (5).

9. The support device according to any of claims 1 to 6, **characterized in that** the flexible structure (7) includes an eyelet (7a) having in its central part, a passage (14) for receiving and holding by wedging, a medical instrument fixing element (3), this eyelet (7a) having an outer side surface (7₃) provided with the dismountable connection system.

10. The support device according to claim 9, **characterized in that** the eyelet (7a) includes, on its outer side surface (7₃) as a dismountable connection system (8), an annular rib (80) cooperating with a housing (110) of complementary shape arranged in the internal wall (6₁) of the mounting hole.

11. The support device according to claim 9, **characterized in that** the eyelet (7a) includes, on its outer side surface (7₃) as a dismountable connection system (8), a frustoconical portion (81) extending from the upper surface (82) up to a radial wall intended to cooperate with a stop surface (12) of the complementary dismountable connection system (11).

12. The support device according to claim 9, **characterized in that** the eyelet (7a) includes, on its outer side surface (7₃) as a dismountable connection system (8), a screw pitch thread (83) cooperating with a tapping (113) of the through hole (6).

13. The support device according to claim 9, **characterized in that** the eyelet (7a) includes, on its outer side surface (7₃) as a dismountable connection system (8), at least one lug (84) intended to be engaged through a slot (114) communicating with a blocking cavity (115) intended to receive the lug after rotation of the eyelet.

14. Packaging equipment for the sterilization and/or the cleaning of medical instruments (2) with fixing elements (3), **characterized in that** it includes at least one support device (1) in accordance with any of claims 1 to 13.
